# EUROPEAN PATENT APPLICATION

(11) **EP 3 954 760 A1**
(43) Date of publication of application: **16.02.2022**
(21) Application number: 20806297.6
(22) Date of filing: 15.05.2020
(51) Int. Cl.: C12N 5/074, A61K 35/42, A61P 11/08

(54) **CLINICAL-GRADE AUTOLOGOUS BRONCHIAL BASAL CELL, TRANSFUSION FORMULATION, AND PREPARATION PROCESS**

(30) Priority: 16.05.2019 CN 201910407062
(71) Applicant: Regend Therapeutics Co., Ltd., Jiangsu 215000 (CN)
(72) Inventor: ZHANG, Ting, Suzhou, Jiangsu 215000 (CN)
(74) Representative: J A Kemp LLP
(86) International application number: PCT/CN2020/090512
(87) International publication number: WO 2020/228819

(57) **Abstract**

Provided are a clinical-grade autologous bronchial basal cell, a transfusion formulation thereof, and a preparation process. The preparation process comprises the following steps: extracting in vitro active bronchial brush biopsy tissue for digestive treatment, and collecting cells after digestion is terminated; performing plating culture on the digested cells by using a culture plate having trophoblast cells plated thereon, collecting cells, performing amplification culture on the digested cells by using a culture plate having trophoblast cells plated thereon, and when cells grow to cover 50%-90% of the surface area of the culture plate, performing secondary culture; and when secondary culture cells grow to cover 85%-95% of the surface area of a culture dish, digesting and collecting adhered cells, and washing. The prepared bronchial basal cell can be used for repairing damaged lung tissue.

## Description

### TECHNICAL FIELD

The present application relates to the technical field of regenerative medicine, in particular, to a clinical-grade autologous bronchial basal cell, deliverable formulation, and preparation process.

### BACKGROUND

The lung is a complex organ composed of epithelial cells and other types of cells, which plays an important role in respiration. During respiration, particularly when contacting some foreign or intrinsic hazardous substances, such as atmospheric contaminants, bacterial viruses, or toxic substances in the blood, a large amount of lung cells will die and inflammatory responses will be induced, resulting in large-scale damage in the lung tissues. Such large-scale damage tends to induce various pulmonary diseases, such as chronic obstructive pulmonary disease (COPD), bronchiectasis (BE), pulmonary fibrosis, etc.

Chronic obstructive pulmonary disease (COPD) is the common name for a group of chronic airway obstructive diseases that have high morbidity and mortality. The latest statistics show that more than 11% of the Chinese over the age of 40 suffer from different levels of COPD. In other words, there are more than 40 million COPD patients in China, of which more than 600 thousands of people die each year. The central link of COPD pathogenesis is chronic airway inflammation caused by smoking or stimulation due to other harmful gas or inhalation particles. Chronic inflammation causes mucosal swelling, epithelial cell necrosis, gland hyperplasia, mucus increase, twisting and collapse in the bronchus, and alveolar structure would be damaged and lung elastance would be reduced when emphysema is developed.

Clinical manifestations of COPD include chronic cough, expectoration, respiratory difficulties in severe cases, and activity capability decrease, and eventually chronic pulmonary heart disease and chronic respiratory failure would be developed, resulting in loss of working capability and self-care abilities, and even death. In clinical it shows incompletely reversible airway obstruction, and gets worse progressively. Currently, the popular treatment method for COPD in clinical is long-term inhalation of long-acting muscarinic antagonist (LAMA) and/or long-acting β2 agonists plus inhaled corticosteroids (LABA + ICS), supplemented by anti-inflammatory therapy, expectorant treatment, and oxygen therapy. However, the above conventional treatment methods are merely symptomatic treatments that cannot fundamentally solve the damage in pulmonary structures. Lung volume reduction surgery as a treatment for end-stage emphysema is still in an early stage of development, and its indications and efficacy are not unclear, and there is a high risk of complications. Bronchiectasis (BE) is associated with chronic suppurative inflammation and fibrosis in the bronchus and its surrounding lung tissue, causing damage to the muscle and elastic tissue of the bronchial wall, and leading to bronchial deformation and persistent expansion and resulting in irreversible damage in the lung tissue of patients. Bronchiectasis usually has a slow progression and may occur at any age. Typical symptoms comprise chronic cough, cough with a large amount of purulent sputum, and recurrent hemoptysis. The incidence in developing countries is significantly higher than the incidence in developed countries. Current studies show that causes of bronchiectasis include infections, congenital and hereditary diseases, cilia abnormalities, immunodeficiency, foreign matter inhalation, and the like. In China, treatments of bronchiectasis mainly include symptomatic treatments and the use of antibiotics. Such treatments however are single and cannot truly reconstruct the damaged lung tissue structure and thus cannot restore the normal function of the lungs. Pulmonary fibrosis usually refers to the end-stage changes of a large group of lung diseases characterized by fibroblast proliferation and large amounts of extracellular matrix aggregation accompanied by inflammation damage and tissue structural destruction. In other words, normal alveolar tissue is damaged and undergoes abnormal repair, resulting in structural abnormalities. In most pulmonary fibrosis patients, the etiology is unknown. Pulmonary fibrosis seriously affects the respiratory function in a human, with manifestations including dry cough, and progressive dyspnea. With the aggravation of the disease and lung damage, the patient's respiratory function deteriorates, and the survival time is only 3-5 years in most patients. Glucocorticoids and immunosuppressors/cytotoxic drugs were ever used as drugs for treating pulmonary fibrosis, but they failed to show satisfactory effects for mid and late-stage pulmonary fibrosis patients. Pirfenidone and Nintedanib have certain effects in delaying the pulmonary function decline of the patients, but can hardly reverse the disease course and can hardly prevent the occurrence of fibrosis. In summary, there currently lacks an effective treatment for pulmonary fibrosis worldwide.

Currently, the methods adopted in clinical treatments for lung diseases associated with lung injury can only delay the progression of the diseases but cannot truly block or even reverse the progress of the diseases. This is because the existing treatment methods cannot repair damaged lung structures, and therefore cannot block or reverse the progression of these diseases.

Transplantation therapy, including clinically mature organ transplantation, tissue transplantation and emerging cell transplantation, is the only option to treat refractory diseases and end-stage organ failure diseases. However, organ and tissue transplantations have unavoidable disadvantages as follows:
1. There are serious shortages of organ or tissue sources.
2. There are significant immune rejections. Organ or tissue transplantations usually use an allograft, which often leads to various levels of immune rejection reactions. The use of immunosuppressors will increase the risks of various opportunistic infections.
3. It usually requires long surgical time and high technical skills, and is complex in the procedure, etc.

In the field of lung diseases, only no more than 1000 cases of lung transplantation could be carried out each year in China, which is far to meet the large demand of patients. Therefore, cell transplantation therapy has drawn more and more attention in the medical field, especially after hematopoietic cell transplantation, skin cell transplantation, and corneal cell transplantation were successfully used for clinical treatment. In recent years, this technique has been applied to the treatment of other tissue and organ diseases, such as skin cell transplantation for treating burns, liver parenchymal cell transplantation for treating liver failure and other liver metabolic diseases, limbal cell transplantation for treating corneal injury, etc. Cell transplantation therapy involves the use of cells having specific functions. Such cells can be obtained via biological engineering methods, and/or proliferated in vitro, and specially cultured, so that the cells have therapeutic functions including enhancing immunity, killing pathogens and tumor cells, and promoting tissue and organ regeneration and body rehabilitation, etc., thus achieving the purpose of treating diseases.

To better solve the rejection problem in cell transplantation, autologous cell transplantation therapy can better solve the above problems and has the following advantages:
1. Cells are autologous, so there is no immune rejection problem;
2. Transplantation cells are derived from adult tissue and organ, i.e., they are part of the body, so there is no tumor formation risk;
3. Cells have plasticity and have active division and migration capabilities, and may supplement the same type of tissue or cells in apoptosis or necrosis in an appropriate environment;
4. Operation is simple. Transplantation cells do not have a normal profile and anatomical structure of an organ, i.e. not a complete organ but a cell population. The cells are often made into a suspension of cells, so there is no need for anastomosis with blood vessels during transplantation. Transplantation could be achieved through a variety of infusion pathways.
5. There are a variety of therapeutic mechanisms. The cells can undertake repair functions by migrating to a damaged site of tissues and differentiating into normal tissue cells. At the same time, the cells can secrete various anti-inflammatory factors by paracrine action to inhibit the secretion and effecting of pro-inflammatory factors.

In summary, cell transplantation has the advantages of good curative effects, fewer side effects, more individuation, more preciseness, and the like, and is currently applied in treatment studies of various diseases including blood system diseases, tumors, diabetes, cardiovascular diseases, nervous system diseases and the like.

In the prior art, previous studies have found that there is a group of functional bronchial basal cells in humans lung bronchial base. Such functional bronchial basal cells express marker genes Krt5 and p63, have the properties of stem cells, are capable of constantly self-renewing and have the potentials to differentiate into alveolar and bronchial epithelial tissue cells. In the year 2015, Nature, the world's leading academic journal, published a research article describing the regenerative mechanism of the lung epithelium using genetic lineage tracing. In that paper, proliferation, migration, and differentiation of distal bronchial basal cells after influenza virus infection were traced using LacZ driven by Krt5-expression in a mouse model. Krt5-positive distal bronchial basal cells were confirmed to have lung regeneration potential. A subsequent study proved that once Krt5-expressing bronchial basal cells were specifically genetically knocked out in the mouse lung, the pulmonary function cannot be repaired; as a consequence tissue fibrosis occurs. Therefore, it was concluded that keeping sufficient amounts of bronchial basal cells in vivo was necessary to repair the lung and inhibit lung injury. Furthermore, for lung injury mice where lung injury cannot be naturally repaired due to lack of bronchial basal cells, bronchial basal cells transplantation can help recover the morphological structure and function of the lung. At the same time, in the lung injury mice induced by bleomycin, the survival period of the injured mice receiving bronchial basal cells transplantation was significantly increased.

In addition, in a lung injury mouse model induced by influenza virus H1N1, blue LacZ-marked bronchial basal cells were transplanted through the trachea into the mouse lung. Donor cells were observed to extensively integrate to receptor's lung and differentiate to form mature alveoli and bronchial structures. It's worth noting that bronchial basal cells transplantation was a complete failure in healthy mice, which can somehow relatively ensure the safety of the transplantation.

Currently, there are no clinical-grade bronchial basal cells in the prior art.

### SUMMARY

The purpose of the present application is to provide clinical-grade autologous bronchial basal cells, a deliverable formulation thereof, and a preparation process therefor. Specific technical solutions are as follows:
Provided is a preparation process of clinical-grade autologous bronchial basal cells, comprising the following steps:
tissue preparation: getting an ex vivo bronchoscopic brushed-off biopsy tissue;
enzymolysis: digesting the bronchoscopic brushed-off biopsy tissue, and collecting digested cells after terminating the digesting;
plating and amplification culture: getting part of the digested cells to perform primary culture by plating the cells on a culture plate pre-coated with feeder layer cells, collecting the primary cultured cells to perform amplification culture on a culture plate pre-coated with feeder layer cells, followed by passage culture when the cells grow to cover 50%-90% of the surface area of the culture plate;
cell collecting: when passage cells grow to cover 85%-95% of the surface area of the culture plate, digesting and collecting adherent cells, and washing.

In some embodiments, in the step of enzymolysis, the digesting is performed at a temperature of 37°C for a period of 0.5-2 h, with a tissue digestion solution comprising 99 v% of DMEM/F12, 1-20 ng/ml of DNase, 0.1-4 mg/ml of protease Type XIV, and 10-200 ng/ml of trypsin.

In some embodiments, in the step of enzymolysis, said terminating the digesting is performed with a stop buffer comprising 90 v% of DMEM and 10 v% fetal bovine serum (FBS).

In some embodiments, the bronchoscopic brushed-off biopsy tissue is taken from at least one site of the lung lobe.

In some embodiments, the digested cells produced in the step of enzymolysis and the primary cultured cells are put into frozen storage; preferably before the frozen storage, bacterial detection and mycoplasma detection are carried out.

In some embodiments, the frozen storage cells are thawed and used to perform the primary culture and/or amplification culture.

In some embodiments, the primary culture comprises the following steps:
re-suspending the cells to be primary cultured in a culture medium and plating the resulting suspension on a culture plate pre-coated with feeder layer cells, and adding antibiotics into the culture plate; preferably, re-suspending a quarter of the cells to be primary cultured in a culture medium and plating into one well of a culture plate pre-coated with feeder layer cells, and re-suspending the remaining three quarters in a culture medium and plating into another well of the culture plate pre-coated with feeder layer cells (since the total amount of cells in each brush biopsy varies greatly, plating the cells into two wells at different densities can ensure harvesting cultured cells of proper density);
performing culture at 37°C, 4-8% CO₂, replacing the culture medium every other day, and collecting the cells when the cells are grown to form clones and further form colonies wherein 80% or more of the clones contain 40-100 cells and clones of grade A and grade B are observed in three fields of view among randomly selected five fields of view.

In some embodiments, said amplification culture on a culture plate pre-coated with feeder layer cells is performed at 37°C, and meanwhile morphology of the cells is observed and detected to ensure clones of grade A and clones of grade B exceed 50% in randomly observed five fields of view; and wherein in said culture plate pre-coated with feeder layer cells, 50-70% of the culture plate area is pre-coated with feeder layer cells.

In some embodiments, the clones of grade A have a regular and smooth outline, and the clones show clear boundary, and cells in the clones are closely packed and have uniform size; and the clones of grade B have a basically regular outline, and the clones show smooth and clear boundary, and in the clones most of the cells are closely packed and have uniform size but a small amount of the cells have a slightly larger size and are slightly loose packed.

In some embodiments, the passage culture comprises n passages (1< n ≤ 7), wherein n-1^{th} passages are carried out with a culture dish pre-coated with feeder layer cells, and when the cells are grown to cover 50-90% of the culture dish surface, the n^{th} passage is performed with a culture dish pre-coated with matrigel.

In some embodiments, each passage of said n passages grows to cover 50% -90% of the culture dish surface.

In some embodiments, the passage culture comprises:
**1^{st} passage**: culturing the cells with a culture dish pre-coated with feeder layer cells, removing supernatant when the cells are grown to cover 50%-90% of the culture dish surface, washing once with 1 × DPBS, adding trypsin for digesting, pipetting up and down for several times to prepare a single-cell suspension when most of the cells become round and bright, terminating the digesting with a stop buffer, collecting cell suspension to perform centrifuging and remove supernatant, re-suspending the cells in a culture medium, and finally plating the suspension into a culture dish pre-coated with feeder layer cells, and replacing the culture medium every other day;
**2^{nd} to n-1^{th} passages**: when the last passage grows to cover 50%-90% of the culture dish surface, removing culture supernatant, washing once with 1× DPBS, adding trypsin for digesting, pipetting up and down for several times to prepare a single-cell suspension when most of the cells become round and bright, terminating the digesting with a stop buffer, collecting a cell suspension to perform centrifuging and remove supernatant, re-suspending the cells in a culture medium, and finally plating the suspension into a culture dish pre-coated with feeder layer cells, and replacing the culture medium every other day; and
**n^{th} passage**: when the last passage grows to cover 50%-90% of the culture dish surface, removing culture supernatant, washing once with 1× DPBS, adding trypsin and 1× DPBS to perform digesting at 37°C for 1-2min, detaching the feeder layer cells by pipetting up and down, again washing once with 1× DPBS, again adding trypsin for digesting, detaching adherent cells by pipetting up and down to prepare a single-cell suspension when most of the cells become round and bright, terminating the digesting with a stop buffer, collecting a cell suspension to perform centrifuging and remove supernatant, re-suspending the cells in a cultural medium, and finally plating the suspension into a culture dish pre-coated with matrigel, and replacing the culture medium every other day.

In some embodiments, after n-2^{th} passages, 1.1 ml of culture supernatant and 3-16×10⁵ cells are collected to carry out tests, wherein bacterial detection and mycoplasma detection give negative results, and biological effect test gives a HOPX positive cell percentage of ≥30%, and cell identification test confirms cells are KRT5 positive and purity analysis shows KRT5 cells percentage is ≥ 90%; and after n-1^{th} passages, 0.8 ml of culture supernatant is taken to carry out endotoxin content detection and streptomycin residue detection, and the results show that endotoxin content < 0.125 Eu/mL, and streptomycin residue < 4 ppb.

In some embodiments, the culture medium has a formulation consisting of: 225 mL of DMEM, 225 mL of F12, 20-70 mL of FBS, 0.2-2 mM of L-glutamine, 1-14 ng/mL of insulin, 0.1-1 ng/mL of epidermal growth factor, 5-30 µg/mL adenine, and 2-20 µg/mL hydrocortisone.

The present application further provides clinical-grade autologous bronchial basal cells prepared by the preparation process as described above.

The present application further provides a deliverable formulation of clinical-grade autologous bronchial basal cells, comprising the clinical-grade autologous bronchial basal cells as described above and a clinical-grade injection liquid.

In some embodiments, the clinical-grade injection liquid is normal saline for injection.

The present application further provides a preparation process of a deliverable formulation of clinical-grade autologous bronchial basal cells, comprising the following steps: washing cells produced by amplification culture for 2-4 times with 1× DPBS, re-suspending the cells in 1× DPBS after the last washing, followed by centrifuging, discarding the supernatant, re-suspending the cell pellet, adding a clinical-grade injection liquid to reach a cell concentration of 1-22 × 10⁶ cells/mL, mixing well, and taking 1 ml of the resulting preparation as a reserved sample.

In some embodiments, after said re-suspending the cells in 1× DPBS, 10 µL of the resulting suspension is taken for trypan blue staining, showing ≥90% cells are alive.

Reagent consumables used in the preparation process of the clinical-grade autologous bronchial basal cells and the deliverable formulation of the present application meet the standards of sterility, mycoplasma free and low endotoxin content.

The technical solutions of the present application have the following advantages:
1. The present application develops clinical-grade cells suitable for lung injury repair for the first time, and achieves industrial preparation of the cells with a suitable preparation process. Bronchial basal cells (2 × 10⁸) that meet the number and quality required by clinical cell treatments can be prepared in a short term (4-8 weeks). The prepared cells are sterile with no mycoplasma contamination, have low streptomycin residue and endotoxin content, and have the potential to differentiate into mature, functional lung cells. 90% or more of the cells express bronchial basal layer cells marker KRT5. Therefore the prepared cells have safety and effectiveness in clinical autologous cell treatments. Clinical practice shows that the bronchial basal cells prepared by this method can differentiate stably after the cells reach the lesion to significantly repair the lung tissue and improve the pulmonary function of patients.
   In addition, bronchial basal cells are prepared into a liquid formulation in order to facilitate clinical cell transplantation. The liquid formulation contains high purity and high-activity bronchial basal cells, which plays a critical role in the success of autologous cell transplantation in clinical applications.
2. The preparation process of the autologous bronchial basal cells and the deliverable formulation is complete, simple and controllable. Frozen storage of the cells provides backups of the cells on the one hand, and allows more flexible and controllable preparation process of the cells and formulations thereof on the other hand. In-process control can enable full-course monitoring of cell quality.
   In the preparation process of the present application, autologous bronchial basal cells are cultured in a specific culture medium and co-cultured with feeder layer cells, thereby ensuring the proliferation activity of the cells and achieving large amounts of proliferation of cells in short term.
3. The deliverable formulation of clinical-grade autologous bronchial basal cells prepared in the present application is expected to fundamentally regenerate the lung structure, repair injured lung tissue and restore pulmonary function in clinical treatments, and thus achieve the purpose of treating pulmonary diseases such as chronic obstructive pulmonary disease, bronchiectasis, interstitial lung disease and the like.

### BRIEF DESCRIPTION OF THE DRAWINGS

In order to more clearly illustrate the embodiments of the present application and the prior art, the drawings used in the embodiments and the prior art will be briefly described below. Obviously, the drawings attached in the following description only represent some examples of the present application, and those skilled in the art can obtain other drawings based on these drawings without any creative intellectual work.
Figure 1 provides a flow chart showing preparation process of deliverable formulation of clinical-grade autologous bronchial basal cells in Example 4 of the present application;
Figure 2 provides a photograph showing morphology of cell clones in Example 4 of the present application;
Figure 3 provides photographs showing typical morphology of cell clones of different grades in Example 4 of the present application, wherein Figure A shows morphology of cell clones of grade A, and Figure B shows morphology of cell clones of grade B;
Figure 4 provide images showing HR-CT results of a bronchiectasis patient before and after application of the deliverable formulation of clinical-grade autologous bronchial basal cells in Experimental Example 2 of the present application;
Figure 5 provides images showing CT results (bullae of the lung) of a patient with interstitial lung disease before and after application of the deliverable formulation of clinical-grade autologous bronchial basal cells in Experimental Example 4 of the present application;
Figure 6 provides images showing CT results (destroyed lung) of a patient with interstitial lung disease before and after application of the deliverable formulation of clinical-grade autologous bronchial basal cells in Experimental Example 4 of the present application.

### DETAILED DESCRIPTION

In the present application, FBS represents fetal bovine serum;
DMEM represents a high glucose DMEM medium;
F12 represents an F12 nutrient mixture;
DPBS represents Dulbecco's Phosphate Buffered Saline;
HOPX represents a marker of the mature, functional lung cells;
KRT5 represents a marker of bronchial basal cells.

### Example 1

Provided is a preparation process of clinical-grade autologous bronchial basal cells, comprising the following steps:
An ex vivo bronchoscopic brushed-off biopsy tissue is selected. Particularly tissues from two different sites of bronchus are selected in this example. Selecting tissues from two or more different sites of bronchus can increase the success rate of separation.

Tissue digestion solution and stop buffer are prepared for later use. The tissue digestion solution comprises 99 v% of DMEM/F12, and remaining is: 1-20 ng/mL of DNase, 0.1-4 mg/mL of protease Type XIV, and 10-200 ng/mL of trypsin. The stop buffer comprises 90 v% of DMEM and 10 v% FBS.

The bronchoscopic brushed-off biopsy tissue is digested with the tissue digestion solution. The digestion is terminated with a stop buffer, and digested cells are collected.

A culture medium is prepared for later use, wherein the culture medium comprises 225 mL of DMEM, 225 mL of F12, 20-70 mL of FBS, 0.2-2 mM L-glutamine, 1-14 ng/mL of insulin, 0.1-1 ng/mL epidermal growth factor, 5-30 µg/mL adenine, and 2-20 µg/mL hydrocortisone.

A culture plate pre-coated with feeder layer cells is prepared for later use. The preparation of the plate is as follows: 20% matrigel is firstly added to coat the culture plate and overspread the bottom surface of the culture plate, and excess liquid is sucked out. Then the culture plate is placed at 37°C for 15 minutes, thereafter irradiation-inactivated feeder layer cells are plated and cultured in a cell incubator (37° C, 5% CO₂). After 1-2 days, the plate is ready for use if the cells spread 50-70% of the bottom surface of the culture plate.

A part of the digested cells are taken, re-suspended in the culture medium and plated into one well of a 6-well culture plate pre-plated with the feeder layer cells. 200 µL of penicillin-streptomycin solution (10000 µ/mL penicillin and 10000 µg/mL streptomycin) is added into each well, followed by incubation at 37°C and 4-8% CO₂. The culture medium is replaced every other day. The cells are collected when the cells are grown to form clones and further form colonies wherein 80% or more of the clones contain 40-100 cells and clones of grade A and grade B are observed in three fields of view among randomly selected five fields of view.

Generally cell clones appear after 4-7 days. The cell clones can be classified as grades A, B, and C depending on the morphology. The clones of grade A have a regular and smooth outline, and the clones show clear boundary, and cells in the clones are closely packed and have uniform size. The clones of grade B have a basically regular outline, and the clones show smooth and clear boundary, and in the clones most of the cells are closely packed and have uniform size but a small amount of the cells have a slightly larger size and are slightly loose packed. The clones of grade C have an irregular outline and unclear boundary, and the cells in the clones are loosely packed and have large size.

If there are very few cell clones, five fields of view are observed under a 10-fold microscope, and if there are no clones of grade A or B in three of the five fields of view, the cells in the well need to be detached via digestion and plated onto the feeder layer cells previously prepared the day before at a ratio of 1: 1 to perform further culture.

The collected cells are cultured in a 6 cm culture dish pre-plated with feeder layer cells. When the cells are grown to cover 50% -90% of the culture dish surface, first passage is carried out as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minute. Supernatant is removed, and the cells are re-suspended in the culture medium. Finally the resulting suspension is plated onto one 6cm culture dish at a density of 5-10×10⁵ cells. The culture medium is replaced every other day.

2nd passage is carried out when the first passage grows to cover 50%-90% of the culture dish surface. The operation is as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached by pipetting up and down and prepared into a single-cell suspension when most of the cells become round and bright. The digestion is terminated with 1 mL of stop buffer, and the resulting cell suspension is collected and centrifuged at 1200 rpm for 3 minutes. Supernatant is removed, and the cells are re-suspended in culture medium. 1.1 mL supernatant and 3-16×10⁵ cells are collected to carry out tests, wherein bacterial detection and mycoplasma detection give negative results, and biological effect test gives a HOPX positive cell percentage of ≥30%, and cell identification test confirms cells are KRT5 positive, and purity analysis shows KRT5 cell percentage is ≥ 90%. Meanwhile the re-suspended cells are plated into four 10cm culture dishes pre-coated with feeder layer cells at 5-20×10⁵ cells per dish, and culture medium is replaced every other day. 0.8 mL culture supernatant of passage 2 is collected to carry out endotoxin content detection and streptomycin residue detection, and the results show that endotoxin content < 0.125 Eu/mL, and streptomycin residue < 4 ppb.

3rd passage is carried out when passage 2 grows to cover 50%-90% of the culture dish surface. The operation is as follows: Supernatant is removed, and the cells are washed once with 1× DPBS, digested with 2 mL of 0.05% (mass/volume ratio: 0.05g/100mL) trypsin at 37°C for 1-2 minutes. The feeder layer cells are detached from the plate by pipetting up and down, again washed with 1× DPBS, and digested with 2 mL of 0.25% (mass/volume ratio: 0.05g/100mL) trypsin. Adherent cells are detached from the plate by pipetting up and down and prepared into a single-cell suspension when most of the cells become round and bright. The digestion is terminated with a stop buffer. A cell suspension is collected and centrifuged at 1200 rpm for 5 minutes, supernatant is removed, and the cells are re-suspended in culture medium. The resulting suspension is plated into twelve 10cm culture dishes pre-coated with matrigel, and culture medium is replaced every other day.

Wherein, the culture dishes pre-coated with matrigel are prepared as follows: 20% matrigel is firstly added to coat the culture plate and overspread the bottom surface of the culture plate, and excess liquid is sucked out. Then the culture plate is placed at 37°C for 15 minutes.

When the 3rd passage grows to cover 85%-95% of the culture dish surface, each culture dish is washed once with 1× DPBS. 2 mL of 0.25% (mass/volume ratio: 0.05g/100mL) trypsin is added, and the dish is placed at 37 °C for 5-15 min. Adherent cells are gently detached from the dish by pipetting up and down with a 1 mL pipette and prepared into a single-cell suspension when most of the cells become round and bright. The digestion is terminated with 2 mL of stop buffer, and a cell suspension is collected. Then the 10cm culture dish is washed with 2 mL of stop buffer and all cell suspensions are collected. Since there is a large amount of cells to be processed in the present step, no more than five culture dishes are operated at the same time, and the collected cell suspensions are temporarily stored at 2-8°C before all the dishes are processed. After all the cell suspensions of this batch are collected, the suspension is centrifuged at 1200 rpm for 5 min, supernatant is removed, and the cells are washed 2-4 times with 1× DPBS. The washing operation is as follows: re-suspended the centrifuged cell pellet with 40mL of 1×DPBS, and centrifuged at 1200 rpm for 5 min, and supernatant is removed.

### Example 2

Provided is a preparation process of clinical-grade autologous bronchial basal cells, comprising the following steps:
An ex vivo bronchoscopic brushed-off biopsy tissue is selected. Particularly tissues from two different sites of bronchus are selected in this example. Selecting tissues from two or more different sites of bronchus can increase the success rate of separation.

Tissue digestion solution and stop buffer are prepared for later use. The tissue digestion solution comprises 99 v% of DMEM/F12, and remaining is: 1-20 ng/mL of DNase, 0.1-4 mg/mL of protease Type XIV, and 10-200 ng/mL of trypsin. The stop buffer comprises 90 v% of DMEM and 10 v% FBS.

The bronchoscopic brushed-off biopsy tissue is digested with the tissue digestion solution. The digestion is terminated with a stop buffer, and digested cells are collected. Bacterial detection and mycoplasma detection are carried out for the digested cells, and then part of the digested cells are put into frozen storage.

The digested cells in frozen storage are recovered. The operation is as follows: The digested cells in frozen storage are rapidly thawed in a 37°C water bath, and all of the resulting liquid is collected in a 15 mL tube, into which 2 mL of stop buffer previously warmed at 37°C is added dropwise. The tube is centrifuged at 1200 rpm for 3 min, supernatant is removed, and the cells are re-suspended in culture medium to prepare a suspension.

A culture medium is prepared for later use, wherein the culture medium comprises 225 mL of DMEM, 225 mL of F12, 20-70 mL of FBS, 0.2-2 mM L-glutamine, 1-14 ng/mL of insulin, 0.1-1 ng/mL epidermal growth factor, 5-30 µg/mL adenine, and 2-20 µg/mL hydrocortisone.

A culture plate pre-coated with feeder layer cells is prepared for later use. The preparation of the plate is as follows: 20% matrigel is firstly added to coat the culture plate and overspread the bottom surface of the culture plate, and excess liquid is sucked out. Then the culture plate is placed at 37°C for 15 minutes, thereafter irradiation-inactivated feeder layer cells are plated and cultured in a cell incubator (37° C, 5% CO₂). After 1-2 days, the plate is ready for use if the cells spread 50-70% of the bottom surface of the culture plate.

A quarter of the previously suspension of the recovered digested cells is re-suspended in the culture medium and plated into one well of a 6-well culture plate pre-plated with the feeder layer cells, and the remaining three quarters are re-suspended in culture medium and plated into another well of the 6-well culture plate pre-plated with the feeder layer cells. 200 uL of penicillin-streptomycin solution (10000 µ/mL penicillin and 10000 µg/mL streptomycin) is added into each well, followed by incubation at 37°C and 4-8% CO₂. The culture medium is replaced every other day. The cells are collected when the cells are grown to form clones and further form colonies wherein 80% or more of the clones contain 40-100 cells and clones of grade A and grade B are observed in three fields of view among randomly selected five fields of view.

Generally cell clones appear after 4-7 days. The cell clones can be classified as grades A, B, and C depending on the morphology. The clones of grade A have a regular and smooth outline, and the clones show clear boundary, and cells in the clones are closely packed and have uniform size. The clones of grade B have a basically regular outline, and the clones show smooth and clear boundary, and in the clones most of the cells are closely packed and have uniform size but a small amount of the cells have a slightly larger size and are slightly loose packed. The clones of grade C have an irregular outline and unclear boundary, and the cells in the clones are loosely packed and have large size.

If there are very few cell clones, five fields of view are observed under a 10-fold microscope, and if there are no clones of grade A or B in three of the five fields of view, the cells in the well need to be detached via digestion and plated onto the feeder layer cells previously prepared the day before at a ratio of 1: 1 to perform further culture.

1.1mL of the culture supernatant is collected to perform bacterial detection and mycoplasma detection before the cells are put into frozen storage.

When the cells are grown to cover 50% -90% of the culture dish surface, first passage is carried out as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minute. Supernatant is removed, and the cells are re-suspended in the culture medium. Finally the resulting suspension is plated onto one 6cm culture dish at a density of 5-10×10⁵ cells. The culture medium is replaced every other day.

2nd passage is carried out when the first passage grows to cover 50%-90% of the culture dish surface. The operation is as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minutes. Supernatant is removed, and the cells are re-suspended in culture medium. 1.1 mL supernatant and 3-16×10⁵ cells are collected to carry out tests, wherein bacterial detection and mycoplasma detection give negative results, and biological effect test gives a HOPX positive cell percentage of ≥30%, and cell identification test confirms cells are KRT5 positive, and purity analysis shows KRT5 cell percentage is ≥ 90%. Meanwhile the re-suspended cells are plated into four 10cm culture dishes pre-coated with feeder layer cells at 5-20×10⁵ cells per dish, and culture medium is replaced every other day. 0.8 mL culture supernatant of passage 2 is collected to carry out endotoxin content detection and streptomycin residue detection, and the results show that endotoxin content < 0.125 Eu/mL, and streptomycin residue < 4 ppb.

3rd passage is carried out when passage 2 grows to cover 50%-90% of the culture dish surface. The operation is as follows: Supernatant is removed, and the cells are washed once with 1× DPBS, digested at 37°C for 1-2 minutes with 2 mL of 0.05%

(mass/volume ratio: 0.05g/100mL) trypsin supplemented with 1.6mL of 1× DPBS. The feeder layer cells are detached from the plate by pipetting up and down, again washed with 1× DPBS, and digested with 2 mL of 0.25% (mass/volume ratio: 0.05g/100mL) trypsin. Adherent cells are detached from the plate by pipetting up and down and prepared into a single-cell suspension when most of the cells become round and bright. The digestion is terminated with a stop buffer. A cell suspension is collected and centrifuged at 1200 rpm for 5 minutes, supernatant is removed, and the cells are re-suspended in culture medium. The resulting suspension is plated into twelve 10cm culture dishes pre-coated with matrigel, and culture medium is replaced every other day.

Wherein, the culture dishes pre-coated with matrigel are prepared as follows: 20% matrigel is firstly added to coat the culture plate and overspread the bottom surface of the culture plate, and excess liquid is sucked out. Then the culture plate is placed at 37°C for 15 minutes.

When the 3rd passage grows to cover 85%-95% of the culture dish surface, each culture dish is washed once with 1× DPBS. 2 mL of 0.25% (mass/volume ratio: 0.05g/100mL) trypsin is added, and the dish is placed at 37 °C for 5-15 min. Adherent cells are gently detached from the dish by pipetting up and down with a 1 mL pipette and prepared into a single-cell suspension when most of the cells become round and bright. The digestion is terminated with 2 mL of stop buffer, and a cell suspension is collected. Then the 10cm culture dish is washed with 2 mL of stop buffer and all cell suspensions are collected. Since there is a large amount of cells to be processed in the present step, no more than five culture dishes are operated at the same time, and the collected cell suspensions are temporarily stored at 2-8°C before all the dishes are processed. After all the cell suspensions of this batch are collected, the suspension is centrifuged at 1200 rpm for 5 min, supernatant is removed, and the cells are washed 2-4 times with 1× DPBS. The washing operation is as follows: re-suspended the cell pellet with 40mL of 1×DPBS, and centrifuged at 1200 rpm for 5 min, and supernatant is removed.

### Example 3

Provided is a preparation process of clinical-grade autologous bronchial basal cells, comprising the following steps:
An ex vivo bronchoscopic brushed-off biopsy tissue is selected. Particularly tissues from two different sites of bronchus are selected in this example. Selecting tissues from two or more different sites of bronchus can increase the success rate of separation.

Tissue digestion solution and stop buffer are prepared for later use. The tissue digestion solution comprises 99 v% of DMEM/F12, and remaining is: 1-20 ng/mL of DNase, 0.1-4 mg/mL of protease Type XIV, and 10-200 ng/mL of trypsin. The stop buffer comprises 90 v% of DMEM and 10 v% FBS.

The bronchoscopic brushed-off biopsy tissue is digested with the tissue digestion solution. The digestion is terminated with a stop buffer, and digested cells are collected. Bacterial detection and mycoplasma detection are carried out for the digested cells, and then part of the digested cells are put into frozen storage.

A culture medium is prepared for later use, wherein the culture medium comprises 225 mL of DMEM, 225 mL of F12, 20-70 mL of FBS, 0.2-2 mM L-glutamine, 1-14 ng/mL of insulin, 0.1-1 ng/mL epidermal growth factor, 5-30 µg/mL adenine, and 2-20 µg/mL hydrocortisone.

A culture plate pre-coated with feeder layer cells is prepared for later use. The preparation of the plate is as follows: 20% matrigel is firstly added to coat the culture plate and overspread the bottom surface of the culture plate, and excess liquid is sucked out. Then the culture plate is placed at 37°C for 15 minutes, thereafter irradiation-inactivated feeder layer cells are plated and cultured in a cell incubator (37° C, 5% CO₂). After 1-2 days, the plate is ready for use if the cells spread 50-70% of the bottom surface of the culture plate.

A part of the digested cells are taken to perform the following steps: A quarter of the cells are re-suspended in the culture medium and plated into one well of a 6-well culture plate pre-plated with the feeder layer cells, and the remaining three quarters are re-suspended in culture medium and plated into another well of the 6-well culture plate pre-plated with the feeder layer cells. 200 µL of penicillin-streptomycin solution (10000 µ/mL penicillin and 10000 µg/mL streptomycin) is added into each well, followed by incubation at 37°C and 4-8% CO₂. The culture medium is replaced every other day. The cells are collected when the cells are grown to form clones and further form colonies wherein 80% or more of the clones contain 40-100 cells and clones of grade A and grade B are observed in three fields of view among randomly selected five fields of view.

Generally cell clones appear after 4-7 days. The cell clones can be classified as grades A, B, and C depending on the morphology. The clones of grade A have a regular and smooth outline, and the clones show clear boundary, and cells in the clones are closely packed and have uniform size. The clones of grade B have a basically regular outline, and the clones show smooth and clear boundary, and in the clones most of the cells are closely packed and have uniform size but a small amount of the cells have a slightly larger size and are slightly loose packed. The clones of grade C have an irregular outline and unclear boundary, and the cells in the clones are loosely packed and have large size.

If there are very few cell clones, five fields of view are observed under a 10-fold microscope, and if there are no clones of grade A or B in three of the five fields of view, the cells in the well need to be detached via digestion and plated onto the feeder layer cells previously prepared the day before at a ratio of 1: 1 to perform further culture.

1.1mL of the culture supernatant is collected to perform bacterial detection and mycoplasma detection before the cells are put into frozen storage. Following steps are conducted if the detection results are satisfactory.

When there is a manufacturing order, the frozen cells are taken out, one tube for cells from each site. The frozen cells are rapidly thawed in a 37°C water bath, and all of the resulting liquid is collected in a 15 mL tube, into which 2 mL of stop buffer previously warmed at 37°C is added dropwise. The tube is centrifuged at 1200 rpm for 3 minutes, supernatant is removed, and the cells are re-suspended in culture medium and cultured in a 6-well culture plate pre-coated with feeder layer cells. During the culture, morphology of the cell clones is observed and monitored. The qualification standard is that: clones of grade A and clones of grade B exceed 50% in five randomly observed fields of view. The following steps are conducted when the quality standard is met. In said culture plate pre-coated with feeder layer cells, 50-70% of the culture plate area is pre-coated with feeder layer cells.

When the thawed cells are grown to cover 50% -90% of the culture dish surface, first passage is carried out as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minute. Supernatant is removed, and the cells are re-suspended in the culture medium. Finally the resulting suspension is plated onto one 6cm culture dish at a density of 5-10×10⁵ cells. The culture medium is replaced every other day.

2nd passage is carried out when the first passage grows to cover 50%-90% of the culture dish surface. The operation is as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minutes. Supernatant is removed, and the cells are re-suspended in culture medium. 1.1 mL supernatant and 3-16×10⁵ cells are collected to carry out tests, wherein bacterial detection and mycoplasma detection give negative results, and biological effect test gives a HOPX positive cell percentage of ≥30%, and cell identification test confirms cells are KRT5 positive, and purity analysis shows KRT5 cell percentage is ≥ 90%. Meanwhile the re-suspended cells are plated into three 10cm culture dishes pre-coated with feeder layer cells at 5-20×10⁵ cells per dish, and culture medium is replaced every other day. 0.8 mL culture supernatant of passage 2 is collected to carry out endotoxin content detection and streptomycin residue detection, and the results show that endotoxin content < 0.125 Eu/mL, and streptomycin residue < 4 ppb.

3rd passage is carried out when passage 2 grows to cover 50%-90% of the culture dish surface. The operation is as follows: Supernatant is removed, and the cells are washed once with 1× DPBS, digested with 2 mL of 0.05% (mass/volume ratio: 0.05g/100mL) trypsin at 37°C for 1-2 minutes. The feeder layer cells are detached from the plate by pipetting up and down, again washed with 1× DPBS, and digested with 2 mL of 0.25% (mass/volume ratio: 0.05g/100mL) trypsin. Adherent cells are detached from the plate by pipetting up and down and prepared into a single-cell suspension when most of the cells become round and bright. The digestion is terminated with a stop buffer. A cell suspension is collected and centrifuged at 1200 rpm for 5 minutes, supernatant is removed, and the cells are re-suspended in culture medium. The resulting suspension is all plated into twelve culture dishes pre-coated with matrigel, and culture medium is replaced every other day.

Wherein, the culture dishes pre-coated with matrigel are prepared as follows: 20% matrigel is firstly added to coat the culture plate and overspread the bottom surface of the culture plate, and excess liquid is sucked out. Then the culture plate is placed at 37°C for 15 minutes.

When the 3rd passage grows to cover 85%-95% of the culture dish surface, each culture dish is washed once with 1× DPBS. 2 mL of 0.25% (mass/volume ratio: 0.05g/100mL) trypsin is added, and the dish is placed at 37 °C for 5-15 min. Adherent cells are gently detached from the dish by pipetting up and down with a 1 mL pipette and prepared into a single-cell suspension when most of the cells become round and bright. The digestion is terminated with 2 mL of stop buffer, and a cell suspension is collected. Then the 10cm culture dish is washed with 2 mL of stop buffer and all cell suspensions are collected. Since there is a large amount of cells to be processed in the present step, no more than five culture dishes are operated at the same time, and the collected cell suspensions are temporarily stored at 2-8°C before all the dishes are processed. After all the cell suspensions of this batch are collected, the suspension is centrifuged at 1200 rpm for 5 min, supernatant is removed, and the cells are washed 2-4 times with 1× DPBS. The washing operation is as follows: re-suspended the cell pellet with 40mL of 1×DPBS, and centrifuged at 1200 rpm for 5 min, and supernatant is removed.

### Example 4

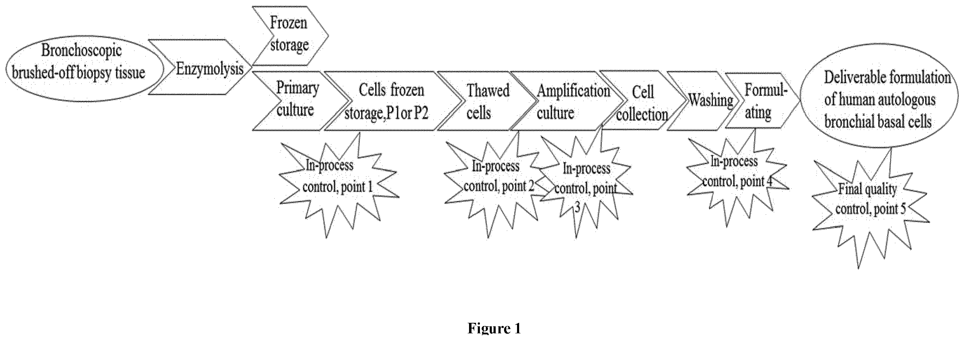

Provided is a preparation process of a deliverable formulation of clinical-grade autologous bronchial basal cells. As shown in figure 1, the preparation process is as follows:
An ex vivo bronchoscopic brushed-off biopsy tissue is selected. Particularly tissues from two different sites of bronchus are selected in this example. Selecting tissues from two or more different sites of bronchus can increase the success rate of separation.

Tissue digestion solution and stop buffer are prepared for later use. The tissue digestion solution comprises 99 v% of DMEM/F12, and remaining is: 1-20 ng/mL of DNase, 0.1-4 mg/mL of protease Type XIV, and 10-200 ng/mL of trypsin. The stop buffer comprises 90 v% of DMEM and 10 v% FBS.

The bronchoscopic brushed-off biopsy tissue is digested with the tissue digestion solution. The digestion is terminated with a stop buffer, and digested cells are collected. Bacterial detection and mycoplasma detection are carried out for the digested cells, and then part of the digested cells are put into frozen storage.

A culture medium is prepared for later use, wherein the culture medium comprises 225 mL of DMEM, 225 mL of F12, 20-70 mL of FBS, 0.2-2 mM L-glutamine, 1-14 ng/mL of insulin, 0.1-1 ng/mL epidermal growth factor, 5-30 µg/mL adenine, and 2-20 µg/mL hydrocortisone.

A culture plate pre-coated with feeder layer cells is prepared for later use. The preparation of the plate is as follows: 20% matrigel is firstly added to coat the culture plate and overspread the bottom surface of the culture plate, and excess liquid is sucked out. Then the culture plate is placed at 37°C for 15 minutes, thereafter irradiation-inactivated feeder layer cells are plated and cultured in a cell incubator (37° C, 5% CO₂). After 1-2 days, the plate is ready for use if the cells spread 50-70% of the bottom surface of the culture plate.

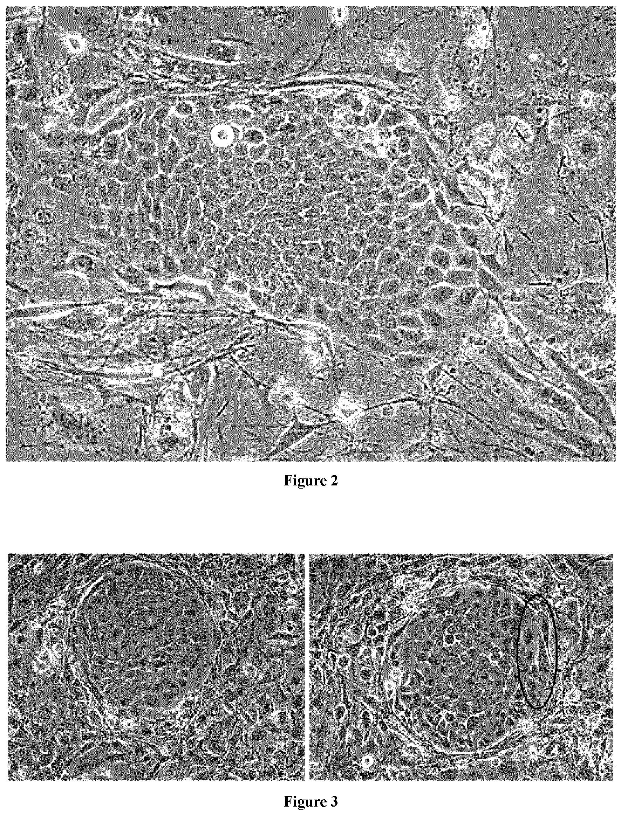

A part of the digested cells are taken to perform the following steps: A quarter of the cells are re-suspended in the culture medium and plated into a 6-well culture plate pre-plated with the feeder layer cells, and the remaining three quarters are re-suspended in culture medium and plated into another 6-well culture plate pre-plated with the feeder layer cells. 200 µL of penicillin-streptomycin solution (10000 µ/mL penicillin and 10000 µg/mL streptomycin) is added into each well, followed by incubation at 37°C and 4-8% CO₂. The culture medium is replaced every other day. When the cells are grown to form clones and further form colonies (as shown in figure 2) wherein 80% or more of the clones contain 40-100 cells and clones of grade A and grade B are observed in three fields of view among five randomly selected fields of view (morphologies of clones of grade A and grade B are respectively as shown in figure 3-A and figure 3-B, wherein larger sized cells are circled), the cells are collected and are put into frozen storage according to sampling sites of bronchoscopic brushed-off biopsy tissue.

Generally cell clones appear after 4-7 days. The cell clones can be classified as grades A, B, and C depending on the morphology. The clones of grade A have a regular and smooth outline, and the clones show clear boundary, and cells in the clones are closely packed and have uniform size. The clones of grade B have a basically regular outline, and the clones show smooth and clear boundary, and in the clones most of the cells are closely packed and have uniform size but a small amount of the cells have a slightly larger size and are slightly loose packed. The clones of grade C have an irregular outline and unclear boundary, and the cells in the clones are loosely packed and have large size.

If there are very few cell clones, five fields of view are observed under a 10-fold microscope, and if there are no clones of grade A or B in three of the five fields of view, the cells in the well need to be detached via digestion and plated onto the feeder layer cells previously prepared the day before at a ratio of 1: 1 to perform further culture. The cells are collected and put into frozen storage when the cells are grown to form clones and further form colonies wherein 80% or more of the clones are grown to contain 40-100 cells. The culture supernatant is collected to perform bacterial detection and mycoplasma detection (In-process control, point 1) before the cells are put into frozen storage. The following steps are performed if the detection results are qualified.

When there is a manufacturing order, the frozen cells are taken out, one tube for cells from each sampling site. The frozen cells are rapidly thawed in a 37°C water bath, and all of the resulting liquid is collected in a 15 mL tube, into which 2 mL of stop buffer previously warmed at 37°C is added dropwise. The tube is centrifuged at 1200 rpm for 3 minutes, supernatant is removed, and the cells are re-suspended in culture medium and cultured on feeder layer cells prepared the day before (in one well of 6 wells). Observe every day and replace the culture medium every other day. Feeder layer cells coats 50-70% of the culture plate area. During the culture, morphology of the cell clones is observed and monitored. The qualification standard is that: clones of grade A and clones of grade B exceed 50% in five randomly observed fields of view. The following steps are conducted when the qualification standard is met.

When the thawed cells are grown to cover 50% -90% of the culture dish surface, first passage is carried out as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minute. Supernatant is removed, and the cells are re-suspended in the culture medium. Finally the resulting suspension is plated onto one 6cm culture dish at a density of 5-10×10⁵ cells. The culture medium is replaced every other day.

2nd passage is carried out when the first passage grows to cover 50%-90% of the culture dish surface. The operation is as follows: Culture supernatant is removed, and the cells are washed once with 1× DPBS, then digested with 1 mL of 0.25% (mass/volume ratio: 0.25g/100mL) trypsin at 37°C for 5-10 minutes. Adherent cells are detached from the plate by pipetting up and down and prepared into a single-cell suspension when most of the cells become round and bright. The digestion is terminated with 1 mL of stop buffer, and the resulting cell suspension is collected and centrifuged at 1200 rpm for 3 minutes. Supernatant is removed, and the cells are re-suspended in culture medium. 1.1 mL supernatant and 3-16×10⁵ cells are collected to carry out tests, wherein bacterial detection and mycoplasma detection give negative results, and biological effect test gives a HOPX positive cell percentage of ≥30%, and cells identification test confirms cells are KRT5 positive, and purity analysis shows KRT5 cell percentage is ≥ 90% (In-process control, point 2). Meanwhile the re-suspended cells are plated into three 10cm culture dishes pre-coated with feeder layer cells at 5-20×10⁵ cells per dish, and culture medium is replaced every other day. 0.8 mL culture supernatant of passage 2 is collected to carry out endotoxin content detection and streptomycin residue detection, and the results show that endotoxin content < 0.125 Eu/mL, and streptomycin residue < 4 ppb (In-process control, point 3).

3rd passage is carried out when passage 2 grows to cover 50%-90% of the culture dish surface. The operation is as follows: Supernatant is removed, and the cells are washed once with 1× DPBS, digested with 2 mL of 0.05% (mass/volume ratio: 0.05g/100mL) trypsin at 37°C for 1-2 minutes. The feeder layer cells are detached from the plate by pipetting up and down, again washed with 1× DPBS, and digested with 2 mL of 0.25% (mass/volume ratio: 0.05g/100mL) trypsin. Adherent cells are detached from the plate by pipetting up and down and prepared into a single-cell suspension when most of the cells become round and bright. The digestion is terminated with a stop buffer. A cell suspension is collected and centrifuged at 1200 rpm for 5 minutes, supernatant is removed, and the cells are re-suspended in culture medium. The resulting suspension is all plated into twelve 10cm culture dishes pre-coated with matrigel, and culture medium is replaced every other day.

Wherein, the culture dishes pre-coated with matrigel are prepared as follows: 20% matrigel is firstly added to coat the culture plate and overspread the bottom surface of the culture plate, and excess liquid is sucked out. Then the culture plate is placed at 37°C for 15 minutes.

When the 3rd passage grows to cover 85%-95% of the culture dish surface, each culture dish is washed once with 1× DPBS. 2 mL of 0.25% (mass/volume ratio: 0.05g/100mL) trypsin is added, and the dish is placed at 37 °C for 5-15 min. Adherent cells are gently detached from the dish by pipetting up and down with a 1 mL pipette and prepared into a single-cell suspension when most of the cells become round and bright. The digestion is terminated with 2 mL of stop buffer, and a cell suspension is collected. Then the 10cm culture dish is washed with 2 mL of stop buffer and all cell suspensions are collected. Since there is a large amount of cells to be processed in the present step, five culture dishes are operated at the same time, and the collected cell suspensions are temporarily stored at 2-8°C before all the dishes are processed. After all the cell suspensions of this batch are collected, the suspension is centrifuged at 1200 rpm for 5 min, supernatant is removed.

The cell pellet are re-suspended in 45mL 1×DPBS. All of the resulting cell suspension is collected into a 50mL centrifuge tube, and centrifuged at 1200 rpm for 5 minutes. Supernatant is removed. The cell pellet are re-suspended in 45mL of 1×DPBS, mixed well by inverting the centrifuge tube upside down. 10uL of the resulting cell suspension is collected for trypan blue staining, showing ≥ 90% cells are alive (In-process control, point 4). The cells in the centrifuge tube are centrifuged at 1200rpm for 5minutes, and supernatant is discarded. The cell pellets are re-suspended in 15mL of normal saline to reach a cell concentration of 1-22×10⁶/mL. 1 mL of the resulting cell preparation is collected as a reserved sample. The appearance test shows that the deliverable formulation is a colorless or white cell suspension without obvious visible impurities (Final quality control, point 5).

The above mentioned bacterial detection is carried out with a plate culture method. Mycoplasma detection is carried out with PCR method.

Endotoxin detection is carried out with Limulus Amoebocyte Lysate (LAL) Gel-Clot method.

Streptomycin residue detection is carried out with competitive enzyme-linked immunosorbent assay (ELISA).

Biological effect test is carried out with in vitro differentiation culture method, and the cells are immuno-stained with a HopX antibody after differentiation.

Cell identification and cell purity detection are carried out with immunofluorescence staining method.

Detection of proportions of alive cells is carried out with Trypan blue staining method.

### Experiment Example 1

A deliverable formulation of clinical-grade autologous bronchial basal cells for lung tissue regeneration medicine is stock-out and transported within 12 h by means of 2-8 °C of cold chain transportation into a hospital for treatment, and then delivered back to a subject through a bronchoscope. The specific steps are as follows:
(1) When the subject has a transcutaneous oxygen saturation of 92% or more and the vital signs are stable, the deliverable formulation of clinical-grade autologous bronchial basal cells is injected into the lobar or segmental bronchi of the lesion site through a bronchofibroscope.
(2) After the deliverable formulation of the clinical-grade autologous bronchial basal cells is delivered, the drug delivery tube is firstly taken out, and then the bronchofibroscope is taken out. If necessary, non-invasive continuous positive airway pressure (CPAP) therapy can be applied in order to assist bronchial basal cell attachment to the lung tissue. Fasting for solid food and liquids are required within 2 hours postoperation. The subject shall avoid coughing as much as possible, and codeine can be administered orally if necessary.
(3) After the delivery is completed, the subject is required to stay in a supine position for about two hours to ensure tight adhesion between the delivered cells and lung injury areas.

### Experimental Example 2

YE, female, 43 years old, suffering from bronchiectasis and emphysema, accompanied by suspected old pulmonary tuberculosis. The treatment included two stages. First stage was to prepare a deliverable formulation of clinical-grade autologous bronchial basal cells, and second stage was to deliver the deliverable formulation of clinical-grade autologous bronchial basal cells back to the lung.
(1) Preparation of a deliverable formulation of clinical-grade autologous bronchial basal cells: YE received bronchoscope examination in September 2016 and trace amounts of brushed-off biopsy tissue were obtained by bronchoscopic brush biopsy. The formulation of clinical-grade autologous bronchial basal cells was prepared according to the preparation process as described in Example 4.
(2) Delivery of the deliverable formulation of clinical-grade autologous bronchial basal cells back to the lung

In April of 2016, the patient received treatment of the above prepared deliverable formulation of clinical-grade autologous bronchial basal cells according to the procedures as described in Experimental Example 1 in the Southwest Hospital of AMU, China.

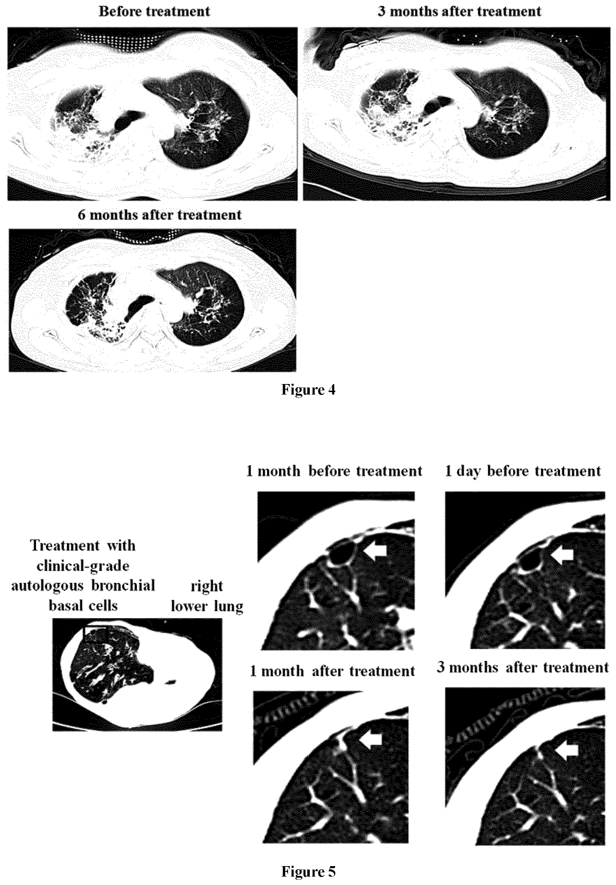

From date of cell delivery to August of 2018, the subject did not exhibit adverse reactions associated with the formulation. Pulmonary function tests showed that, one month after cell delivery, the forced vital capacity/predicted (FVC%), which is an index of lung ventilation capacity, was increased to 58.0% from 49.5% at baseline. Three months later the FVC% was further increased to 63.6% and maintained for one year post-treatment. The diffusion capacity of carbon monoxide/predicted (DLCO%) was also increased from 56.3% before treatment to 68.2% one year post-treatment. The result of 6-minute walk test (6MWT) of the patient was increased from 447 meters before treatment to 495 meters post-treatment (Table 1). The patient reported that she could climb up to the 5^{th} floor after the treatment, while she could only climb up to the 1^{st} or 2^{nd} floor before the treatment. Symptoms, such as shortness of breath, wheeziness and coughing, were also greatly ameliorated. More importantly, comparison of HR-CT results of the subject before and after cell delivery showed that, new lung structures were obviously generated at the destroyed lesions of the right lung as the time goes after the treatment, so the structural damage in the patient's lung was repaired (Figure 4). This is consistent with previously described improvements in the patient's pulmonary function test results, as well as obvious relief of the various symptoms, confirming the treatment with the deliverable formulation of clinical-grade autologous bronchial basal cells is effective.

**Table 1. Results of pulmonary function tests and 6MWT of the patient before and after treatment**

| **Detection index** | **Before treatment** | **1 month after treatment** | **3 months after treatment** | **6 months after treatment** | **1 year after treatment** |
|---|---|---|---|---|---|
| VC MAX% | 54.7 | 57.1 | 63.9 | 63.9 | 63.9 |
| FEV1% | 41.1 | 34.4 | 41.8 | 38.9 | 45.8 |
| FVC% | 49.5 | 58.0 | 63.6 | 63.5 | 65 |
| TLCO-SB% | 56.3 | 57.1 | 61.8 | 67.3 | 68.2 |
| 6MWT (meter) | 447 | 495 | 540 | 423 | 495 |

### Experimental Example 3

In 2016, a subject with chronic obstructive pulmonary disease (chronic bronchitis) received treatment with the deliverable formulation of clinical-grade autologous bronchial basal cells in the Southwest Hospital of AMU, China. The treatment was divided into two stages, wherein the first stage was to prepare a deliverable formulation of clinical-grade autologous bronchial basal cells, and the second stage was to deliver the deliverable formulation of clinical-grade autologous bronchial basal cells back to the subject's lung.
(1) A deliverable formulation of clinical-grade autologous bronchial basal cells was prepared according to the preparation process as described in Example 4.
(2) The deliverable formulation of clinical-grade autologous bronchial basal cells was delivered back to the patient.

The patient received treatment of the above prepared deliverable formulation of clinical-grade autologous bronchial basal cells according to the procedures as described in Experimental Example 1. Half year after the treatment, the subject exhibited no adverse reactions associated with the formulation, while the subject's pulmonary function indexes, such as FEV1, FEV1/FVC, DLCO, MMEF, MVV, etc. were significantly increased, and the symptoms of the subject evaluated by the St George' Respiratory Questionnaire (SGRQ) were significantly improved (Table 2).

**Table 2. Results of the pulmonary function tests, 6MWT, and SGRQ score of the subject before and after treatment**

| **Detection index** | **Before treatment** | **1 month after treatment** | **3 months after treatment** | **Half year after treatment** |
|---|---|---|---|---|
| FEV1% | 31.7 | 27.8 | 37.8 | 41.0 |
| FVC% | 53.7 | 54.8 | 62.0 | 67.7 |
| MMEF75/25% | 10.9 | 9.4 | 13.0 | 14.1 |
| TLCO-SB% | 37.6 | 93.9 | 74.8 | 82.0 |
| 6MWT(meter) | 522 | 429 | 570 | 510 |
| SGRQ score | 27 | 30 | 22 | 17 |

### Experimental Example 4

LIANG, male, suffering from interstitial lung disease and CT images showing obvious pulmonary bullae in the right lower lung, received treatment with deliverable formulation of clinical-grade autologous bronchial basal cells in November 2016 in Shanghai East Hospital, China. The treatment was divided into two stages, wherein the first stage was to prepare a deliverable formulation of clinical-grade autologous bronchial basal cells, and the second stage was to deliver the deliverable formulation of clinical-grade autologous bronchial basal cells back to the patient's lung.
(1) A deliverable formulation of clinical-grade autologous bronchial basal cells was prepared according to the preparation process as described in Example 4.
(2) The deliverable formulation of clinical-grade autologous bronchial basal cells was delivered back to the patient.

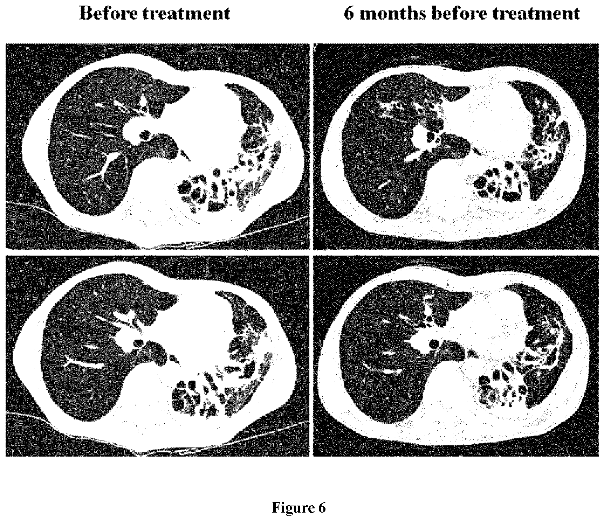

The patient received treatment of the above prepared deliverable formulation of clinical-grade autologous bronchial basal cells according to the procedures as described in Experimental Example 1. CT images at one-month and three-month after the treatment showed significant remission of the pulmonary bullae in the right lower lung (Figure 5). The subject reported evidently relieved symptoms. 6 months after the treatment, all pulmonary function indexes were increased significantly. Acute exacerbation rates were reduced from average 4-5 times per year before the treatment to almost none. CT results at 6 months after the cell delivery showed that, new lung structure was formed in the destroyed area of the left lung, and the original damage of the lung structure was mostly repaired (Figure 6). This is consistent with the improvements in the results of the patient's other indexes and significant alleviation of the symptoms reported by the patient, confirming the treatment of the deliverable formulation of clinical-grade autologous bronchial basal cells is effective. Apparently, the aforementioned embodiments are merely examples illustrated for clearly describing the present invention, rather than limiting the implementation ways thereof. For those skilled in the art, various changes and modifications in other different forms can be made on the basis of the aforementioned description. It is unnecessary and impossible to exhaustively list all the implementation ways herein. However, any obvious changes or modifications derived from the aforementioned description are intended to be embraced within the protection scope of the present invention.

## Claims

1. A preparation process of clinical-grade autologous bronchial basal cells, comprising the following steps:
tissue preparation: getting an ex vivo bronchoscopic brushed-off biopsy tissue;
enzymolysis: digesting the bronchoscopic brushed-off biopsy tissue, and collecting digested cells after terminating the digesting;
plating and amplification culture: getting part of the digested cells to perform primary culture by plating the cells on a culture plate pre-coated with feeder layer cells, collecting the primary cultured cells to perform amplification culture on a culture plate pre-coated with feeder layer cells, followed by passage culture when the cells grow to cover 50%-90% of the surface area of the culture plate;
cell collecting: when passage cells grow to cover 85%-95% of the surface area of the culture plate, digesting and collecting adherent cells, and washing.

2. The preparation process according to claim 1, wherein in the step of enzymolysis, the digesting is performed at a temperature of 37°C for a period of 0.5-2 h, with a tissue digestion solution comprising 99 v% of DMEM/F12, 1-20 ng/ml of DNase, 0.1-4 mg/ml of protease Type XIV, and 10-200 ng/ml of trypsin.

3. The preparation process of claim 1 or 2, wherein in the step of enzymolysis, said terminating the digesting is performed with a stop buffer comprising 90 v% of DMEM and 10 v% of fetal bovine serum.

4. The preparation process according to any one of claims 1-3, wherein the bronchoscopic brushed-off biopsy tissue is taken from at least one site of the lung lobe.

5. The preparation process according to any one of claims 1-3, wherein the digested cells yielded in the step of enzymolysis and the primary cultured cells are put into frozen storage; preferably before the frozen storage, bacterial detection and mycoplasma detection are carried out.

6. The preparation process according to claim 5, wherein the frozen storage cells are thawed and used to perform the primary culture and/or amplification culture.

7. The preparation process according to any one of claims 1-6, wherein the primary culture comprises the following steps:
re-suspending the cells to be primary cultured in a culture medium and plating the resulting suspension on a culture plate pre-coated with feeder layer cells, and adding antibiotics into the culture plate;
performing culture at 37°C, 4-8% CO₂, replacing the culture medium every other day, and collecting the cells when the cells are grown to form clones and further form colonies wherein 80% or more of the clones contain 40-100 cells and clones of grade A and grade B are observed in three fields of view among randomly selected five fields of view.

8. The preparation process according to any one of claims 1-7, wherein said amplification culture on a culture plate pre-coated with feeder layer cells is performed at 37°C, and meanwhile morphology of the cells is observed and detected to ensure clones of grade A and clones of grade B exceed 50% in randomly observed five fields of view; and
wherein in said culture plate pre-coated with feeder layer cells, 50-70% of the culture plate area is pre-coated with feeder layer cells.

9. The preparation process according to claim 7 or 8, wherein the clones of grade A have a regular and smooth outline, and the clones show clear boundary, and cells in the clones are closely packed and have uniform size; and
wherein the clones of grade B have a basically regular outline, and the clones show smooth and clear boundary, and in the clones most of the cells are closely packed and have uniform size but a small amount of the cells have a slightly larger size and are slightly loose packed.

10. The preparation process according to any one of claims 1-9, wherein the passage culture comprises n passages (1< n ≤ 7), wherein n-1^{th} passages are carried out with a culture dish pre-coated with feeder layer cells, and when the cells are grown to cover 50-90% of the culture dish surface, the n^{th} passage is performed with a culture dish pre-coated with matrigel.

11. The preparation process according to claim 10, wherein each passage of said n passages grows to cover 50% -90% of the culture dish surface.

12. The preparation process according to claim 10 or 11, wherein the passage culture comprises:
**1^{st} passage**: culturing the cells with a culture dish pre-coated with feeder layer cells, removing supernatant when the cells are grown to cover 50%-90% of the culture dish surface, washing once with 1 × DPBS, adding trypsin for digesting, pipetting up and down to prepare a single-cell suspension when most of the cells become round and bright, terminating the digesting with a stop buffer, collecting cell suspension to perform centrifuging and remove supernatant, re-suspending the cells in a culture medium, and finally plating the suspension into a culture dish pre-coated with feeder layer cells, and replacing the culture medium every other day;
**2^{nd} to n-1^{th} passages**: when the last passage grows to cover 50%-90% of the culture dish surface, removing culture supernatant, washing once with 1× DPBS, adding trypsin for digesting, pipetting up and down to prepare a single-cell suspension when most of the cells become round and bright, terminating the digesting with a stop buffer, collecting cell suspension to perform centrifuging and remove supernatant, re-suspending the cells in a culture medium, and finally plating the suspension into a culture dish pre-coated with feeder layer cells, and replacing the culture medium every other day; and
**n^{th} passage**: when the last passage grows to cover 50%-90% of the culture dish surface, removing culture supernatant, washing once with 1× DPBS, adding trypsin and 1× DPBS to perform digesting at 37°C for 1-2min, detaching the feeder layer cells by pipetting up and down, again washing once with 1× DPBS, again adding trypsin for digesting, detaching adherent cells by pipetting up and down to prepare a single-cell suspension when most of the cells become round and bright, terminating the digesting with a stop buffer, collecting cell suspension to perform centrifuging and remove supernatant, re-suspending the cells in a cultural medium, and finally plating the suspension into a culture dish pre-coated with matrigel, and replacing the culture medium every other day.

13. The preparation process according to any one of claims 1-12, wherein after n-2^{th} passages, 1.1 ml of culture supernatant and 3-16×10⁵ cells are collected to carry out tests, wherein bacterial detection and mycoplasma detection give negative results, and biological effect test gives a HOPX positive cell percentage of ≥30%, and cell identification test confirms cells are KRT5 positive and purity analysis shows KRT5 cells percentage is ≥ 90%; and
after n-1^{th} passages, 0.8 ml of culture supernatant is taken to carry out endotoxin content detection and streptomycin residue detection, and the results show that endotoxin content < 0.125 Eu/mL, and streptomycin residue < 4 ppb.

14. The preparation process according to any one of claims 1-13, wherein the culture medium has a formulation consisting of: 225 mL of DMEM, 225 mL of F12, 20-70 mL of FBS, 0.2-2 mM of L-glutamine, 1-14 ng/mL of insulin, 0.1-1 ng/mL of epidermal growth factor, 5-30 (µg/mL adenine, and 2-20 (µg/mL hydrocortisone.

15. Clinical-grade autologous bronchial basal cells prepared by the preparation process of any one of claims 1-14.

16. A deliverable formulation of clinical-grade autologous bronchial basal cells, comprising the clinical-grade autologous bronchial basal cells of claim 15 and a clinical-grade injection liquid.

17. A preparation process of a deliverable formulation of clinical-grade autologous bronchial basal cells, comprising the following steps: washing cells produced by amplification culture for 2-4 times with 1× DPBS, re-suspending the cells in 1× DPBS after the last washing, followed by centrifuging, discarding the supernatant, re-suspending the cell pellet, adding a clinical-grade injection liquid to reach a cell concentration of 1-22 × 10⁶ cells/mL, mixing well, and taking 1 ml of the resulting preparation as a reserved sample.

18. The preparation process according to claim 17, wherein the clinical-grade injection liquid is normal saline for injection.

19. The preparation process according to claim 17, wherein after said re-suspending the cells in 1× DPBS, 10 µL of the resulting suspension is taken for trypan blue staining, showing ≥90% cells are alive.
